# EUROPEAN PATENT APPLICATION

(11) **EP 4 400 097 A1**
(43) Date of publication of application: **17.07.2024**
(21) Application number: 22866469.4
(22) Date of filing: 29.08.2022
(51) Int. Cl.: A61K 9/10, A61K 31/496, A61P 25/24, A61P 25/18

(54) **LONG-ACTING BREXPIPRAZOLE PREPARATION FOR INJECTION AND PREPARATION METHOD THEREFOR**

(30) Priority: 07.09.2021 CN 202111043276
(71) Applicant: Sichuan Kelun Pharmaceutical Research Institute Co., Ltd., Chengdu, Sichuan 611138 (CN)
(72) Inventor: LI, Ming, Chengdu, Sichuan 611138 (CN); WEI, Wei, Chengdu, Sichuan 611138 (CN); SU, Zhengxing, Chengdu, Sichuan 611138 (CN); YI, Cong, Chengdu, Sichuan 611138 (CN); LI, Dan, Chengdu, Sichuan 611138 (CN); LIANG, Xiangyong, Chengdu, Sichuan 611138 (CN); KE, Duo, Chengdu, Sichuan 611138 (CN); ZHAO, Dong, Chengdu, Sichuan 611138 (CN); WANG, Jingyi, Chengdu, Sichuan 611138 (CN); LIU, Sichuan, Chengdu, Sichuan 611138 (CN)
(74) Representative: Turner, Craig Robert
(86) International application number: PCT/CN2022/115484
(87) International publication number: WO 2023/036003

(57) **Abstract**

A long-acting brexpiprazole preparation for injection and a preparation method therefor. The preparation method therefor comprises: (a) dissolving brexpiprazole in an organic solvent to form a brexpiprazole solution; (b1) pumping the brexpiprazole solution into a sheared or violently stirred precipitation solvent, so as to precipitate brexpiprazole, or (b2) pumping the precipitation solvent into the sheared or violently stirred brexpiprazole solution, so as to precipitate brexpiprazole; (c) filtering and optionally using water to wash an obtained solid; and (d1) drying the obtained solid, and respectively packaging the dried solid and an auxiliary material solution to obtain the long-acting brexpiprazole preparation for injection, or (d2) dispersing the obtained solid into the auxiliary material solution to obtain the long-acting brexpiprazole preparation for injection.

## Description

### Technical Field

The present invention belongs to the field of pharmaceutical preparations, and relates to a long-acting brexpiprazole preparation and a method for preparing the same. Specifically, the present invention relates to a long-acting brexpiprazole preparation for injection and a method for preparing the same.

### Background Art

Brexpiprazole, a product of iteration of aripiprazole, is a second generation antischizophrenic drug, and has the following structural formula:

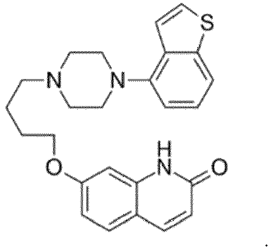

As a partial agonist for the 5-HT1A receptor and the dopamine D2 receptor, and an antagonist for the 5-HT2A receptor, brexpiprazole is clinically used for adjuvant treatment of adult schizophrenia or adult major depression. Compared with aripiprazole, brexpiprazole has lower intrinsic activity for the dopamine D2 receptor, higher binding capacity with the5-HT1 and 5-HT2 receptors, lower extrapyramidal side effects, and lower akathisia adverse effect, and thus is a first-line drug for treating schizophrenia. However, currently marketed products of brexpiprazole are only tablets for oral administration once a day and there are still many problems in clinical use For example, schizophrenia has a relatively long course, and patients are prone to hiding drugs, missing drug taking and the like since the currently marketed brexpiprazole tablets need to be administrated frequently. In order to improve the medication compliance and long-term treatment compliance of the patients, increase the effectiveness of treatment, and avoid relapse and deterioration of disease, it is of great clinical significance to develop a long-acting (>7 days) brexpiprazole preparation.

CN 107536802A discloses an aqueous suspension of brexpiprazole for injection, which can achieve a sustained release of brexpiprazole for at least one week. However, although the aqueous suspension disclosed in CN 107536802Ahas the characteristic of sustained release, its plasma drug concentration test in rats shows that early release of the drug is slow, and a plasma drug concentration fluctuates greatly, rendering it difficult to maintain a gently effective plasma drug concentration. Therefore, its clinical advantage is not significant. In addition, the preparation of this preparation relies on the addition of a particle binder to form secondary particles that are then suspended in an aqueous solution. In the early stage, methods such as ball milling need to be used to reduce particle size to form primary particles. Thus, the preparation method is complicated.

Therefore, there is still a need to provide a long-acting brexpiprazole preparation for injection, which may start to be released quickly and reach an effective therapeutic level after injection (for example, after intramuscular injection), and continue to be released slowly to maintain the effective therapeutic level for 30 days or longer, thereby improving the compliance of a patient to the maximum extent.

The present invention provides a long-acting brexpiprazole preparation for injection having the above advantages and a method for preparing the same. In particular, the preparation method of the present invention is simple and convenient to operate, and the brexpiprazole suspension for injection thus provided has the advantages of controllable particle size, good reproducibility, and good stability.

### Summary of the Invention

In a first aspect, the present invention provides a method for preparing a long-acting brexpiprazole preparation for injection, comprising the following steps:
(a) dissolving brexpiprazole in an organic solvent to form a brexpiprazole solution, wherein the organic solvent is selected from a group consisting of dimethyl sulfoxide, dichloromethane, dichloromethane-methanol, dimethylformamide, dimethylformamide-isopropanol, dimethylformamide-water, tetrahydrofuran-water, tetrahydrofuran-isopropanol, ethanol, and glacial acetic acid-water;
(b1) pumping the brexpiprazole solution into a precipitation solvent under shearing or vigorous stirring to precipitate brexpiprazole; or (b2) pumping a precipitation solvent into the brexpiprazole solution under shearing or vigorous stirring to precipitate brexpiprazole, wherein the precipitation solvent is selected from a group consisting of water, an organic solvent selected from a group consisting of ethanol and dimethyl sulfoxide, and a mixture thereof, and optionally comprises a surfactant selected from a group consisting of Tween 20, Tween 80, Span 20, Span 40, Span 60, Span 80, succinate and glyceryl monostearate;
(c) filtering, and optionally washing a resulting solid with water in an amount of not less than 5 times, preferably 8 to 20 times, and more preferably 10 to 15 times a volume of the brexpiprazole solution in step (b1) or (b2); and
(d1) drying the solid, and packaging the dried solid and an excipient solution separately to obtain the long-acting brexpiprazole preparation for injection; or (d2) dispersing the solid into an excipient solution after drying or directly without drying to obtain the long-acting brexpiprazole preparation for injection.

Specifically, the steps of the method of the present invention are described below.

### Step (a). Dissolving brexpiprazole in an organic solvent to form a brexpiprazole solution

In step (a), the organic solvent may be selected from a group consisting of dimethyl sulfoxide, dichloromethane, dichloromethane-methanol, dimethylformamide, dimethylformamide-isopropanol, dimethylformamide-water, tetrahydrofuran-water, tetrahydrofuran-isopropanol, ethanol, and glacial acetic acid-water. Preferably, the organic solvent is selected from a group consisting of dimethyl sulfoxide, dichloromethane, dichloromethane-methanol, ethanol and glacial acetic acid-water, and more preferably dimethyl sulfoxide.

In step (a), the solubility of brexpiprazole in the organic solvent may be increased by heating and/or stirring, thus facilitating the dissolving process. Preferably, the dissolving is performed with stirring at a temperature of 40°C to 80°C, preferably 50°C to 70°C, more preferably 60°C to 70°C, and more preferably about 60°C to 65°C.

Optionally, nitrogen is introduced for protection during the whole dissolving process of brexpiprazole so as to effectively prevent formation of unknown process impurities.

### Step (b1). Pumping the brexpiprazole solution into a precipitation solvent under shearing or vigorous stirring to precipitate brexpiprazole; or step (b2). Pumping a precipitation solvent into the brexpiprazole solution under shearins or visorous stirring to precipitate brexpiprazole

In step (b1) and step (b2), the precipitation solvent may be water, an organic solvent or a mixture thereof, preferably water or ethanol, or water-ethanol or water-dimethyl sulfoxide in a volume ratio of 20:1 to 1:20, preferably 10:1 to 1:10, and more preferably 5:1 to 1:5.

In step (b1) and step (b2), the precipitation solvent may further comprise a surfactant to facilitate dispersion. The surfactant may be in an amount of 0.1% to 3%, preferably 0.5% to 2%, based on the weight of brexpiprazole in the resulting system after the completion of the pumping. The surfactant includes, but is not limited to, polysorbate 20 (Tween 20), polysorbate 80 (Tween 80), sorbitan fatty acid esters (Span 20, 40, 60 or 80), succinate, and glyceryl monostearate. Preferably, the precipitation solvent comprises Tween 80 in an amount of 0.1% to 3%, preferably 0.5% to 2%, of the weight of brexpiprazole in the resulting system after the completion of the pumping. The surfactant may be contained in the precipitated brexpiprazole precipitate, and may be substantially removed by washing with water in a subsequent step.

In step (b1) and step (b2), the purpose of controlling the particle size of the product may be achieved by, for example, using a peristaltic pump to control the pumping velocity of the solution. The pumping may be performed with a single pump head. In order to accelerate the preparation of a sample, a plurality of pump heads may further be used for simultaneous pumping. Preferably, each pump head is at a pumping velocity of 70 mL/min to 300 mL/min, preferably 70 mL/min to 220 mL/min, and more preferably 100 mL/min to 200 mL/min, such as 150 mL/min.

In step (b1) and step (b2), the volume ratio of the pumped brexpiprazole solution to the precipitation solvent may be 2:1 to 1:10, preferably 2:1 to 1:5, and more preferably 1:1 to 1:4, such as 1:2.

In step (b1) and step (b2), the purpose of controlling the particle size of the product may be achieved by controlling the cooling process. In general, the higher the cooling rate is, the smaller the particle size of the product is. Usually, the particle size of the precipitated sample is controlled by controlling the temperature of the precipitation solvent. In some embodiments of the present invention, the temperature of the precipitation solvent is -20°C to 25°C. More specifically, when water is used as the precipitation solvent, its temperature is 1°C to 25°C; when an organic solvent is used as the precipitation solvent, the temperature of the precipitation solvent is -20°C to 0°C; and when a mixture of water and an organic solvent is used as the precipitation solvent, the temperature of the precipitation solvent is -10°C to 25°C. Preferably, the temperature of the precipitation solvent is 0°C to 20°C.

In step (b1) and step (b2), the particle size of the product may be controlled by regulating the shearing velocity or stirring velocity. For example, the vigorous stirring is at a velocity of 200 rpm to 2000 rpm, preferably 500 rpm to 1000 rpm; and the shearing is at a linear velocity of 1.57 m/s to 15.7 m/s. Particularly, in step (b1), the shearing is at a linear velocity of 1.57 m/s to 15.7 m/s, preferably 1.57 to 4.71 m/s, and in step (b2), the shearing is at a linear velocity of 7.85 m/s to 12.56 m/s.

The term "linear velocity" used herein refers to a velocity at which any point on an object makes a circular motion around a fixed axis, and is generally defined as an instantaneous velocity at which a mass point (or each point on the object) makes a curvilinear motion (including the circular motion). The linear velocity is in a tangential direction of a motion track, and thus is also called a tangential velocity. It is a physical quantity describing the motion velocity and direction of a mass point making a curvilinear motion.

The particle size of the brexpiprazole particles precipitated under the above controlled conditions is controllable within a range of 3 µm to 100 µm. The particle size described herein refers to a particle size corresponding to 50% of volume distribution, i.e., Dv50, which is obtained by a wet method measurement using a MasterSizer 3000 laser diffraction particle size analyzer (Malvern), and is preferably 5 µm to 30 µm, and more preferably 7 µm to 20 µm.

### Step (c). Filtering, and optionally washing a resulting solid with water

In step (c), the resulting solid is separated by filtration, and optionally washed with a great amount of water to remove a small amount of surfactant that may be present therein such that the resulting solid contains no surfactant. The amount of the water used for washing is generally not less than 5 times, preferably 8 to 20 times, and more preferably 10 to 15 times a volume of the brexpiprazole solution in step (b1) or (b2).

Preferably, the resulting solid in step (c) does not contain a particle binder such as sodium chloride.

### Step (d1). Drying the solid, and packaging the dried solid and an excipient solution separately to obtain the long-acting brexpiprazole preparation for injection; or (d2). Dispersing the solid into an excipient solution to obtain the long-acting brexpiprazole preparation for injection

In step (d1), drying may be performed by methods known in the art, including, but not limited to, heat drying or vacuum heat drying; and stirring may be performed during drying, so as to prevent lumping. The sterile raw material obtained after drying may be packaged according to a predetermined dose with a unit dose which may range from 50 mg to 400 mg, and is packaged separately from the excipient solution. Before use, the excipient solution is mixed with the sterile raw material by methods such as shaking, vortexing or ultrasonic treatment, such that the concentration of brexpiprazole is 5 wt% to 40 wt%, preferably 5 wt% to 20 wt%, and more preferably 7 wt% to 17 wt%.

In step (d2), the solid may be dispersed in the excipient solution after drying in the same manner as described in step (d1) or directly without drying. The dispersion may be performed by stirring, shearing, ultrasoic treatment or a combination thereof. A suspension having a concentration of brexpiprazole of 5 wt% to 40 wt%, preferably 5 wt% to 20 wt%, and more preferably 7 wt% to 17 wt% is obtained, and is bottled into products of different specifications.

The above excipient solution may comprise:
1) one or more suspending agents;
2) one or more wetting agents;
3) one or more pH regulators;
4) one or more osmotic pressure regulators; and/or
5) a dispersing agent.

As the suspending agent, those well-known to a person skilled in the art may be used, including, but not limited to, polyethylene glycols (for example, polyethylene glycol 4000), carboxymethylcellulose or a sodium salt thereof, polyvinylpyrrolidone (for example, PVP K12 or PVP K30), hypromellose, methylcellulose, poloxamer (for example, poloxamer 188 or 407) or polyvinyl alcohol. Preferably, the suspending agent is carboxymethylcellulose or a sodium salt thereof. For the method with the above step (d1), the suspending agent is preferably in a concentration of 0.01% to 20% (w/v), more preferably 0.05% to 10% (w/v), and most preferably 0.2% to 5% (w/v), by volume of the excipient solution. For the method with the above step (d2), the suspending agent is preferably in a concentration of 0.01% to 20% (w/v), more preferably 0.05% to 10% (w/v), and most preferably 0.2% to 5% (w/v), by volume of the resulting long-acting brexpiprazole preparation for injection (suspension).

As the wetting agent, common surfactants in the art may be used, including, but not limited to, polysorbates (Tween 20, 40, 60 or 80), sorbitan fatty acid esters (Span 20, 40, 60 or 80), poloxamer, sodium dodecyl sulfate (SDS), fatty acid monoglyceride, polyoxyethylene castor oil or lecithin. Preferably, the wetting agent is Tweens such as Tween 80. For the method with the above step (d1), the suspending agent is preferably in a concentration of 0.01% to 20% (w/v), more preferably 0.05% to 10% (w/v), and most preferably 0.2% to 5% (w/v), by volume of the excipient solution. For the method with the above step (d2), the wetting agent is preferably in a concentration of 0.01% to 20% (w/v), more preferably 0.05% to 10% (w/v), and most preferably 0.1% to 2% (w/v), by volume of the resulting long-acting brexpiprazole preparation for injection (suspension).

As the pH regulator, sodium phosphate, disodium hydrogen phosphate, sodium dihydrogen phosphate, potassium phosphate, dipotassium hydrogen phosphate, potassium dihydrogen phosphate, trometamol, sodium carbonate, sodium acetate, sodium bicarbonate, meglumine, arginine, triethanolamine, citric acid and/or acetic acid may be used. Preferably, the pH regulator is disodium hydrogen phosphate and/or sodium dihydrogen phosphate. Preferably, the pH value of the excipient solution is 4 to 9, more preferably 6 to 8, and most preferably 6.5 to 7.5.

As the osmotic pressure regulator, sodium chloride, glucose and/or mannitol may be used. Preferably, the osmotic pressure regulator is mannitol or sodium chloride.

As the dispersing agent, an aqueous vehicle, preferably water for injection, may be used.

In a second aspect, the present invention provides a long-acting brexpiprazole preparation for injection, which is a suspension, or can be prepared as a suspension by mixing a solid and an excipient solution contained in the preparation, wherein the suspension contains 5 wt% to 40 wt%, preferably 5 wt% to 20 wt%, and more preferably 7 wt% to 17 wt% of brexpiprazole.

In some embodiments, particles in the suspension have a particle size distribution as follows: not less than 3% of the particles have a particle size of smaller than 5 µm, and not less than 15% of the particles have a particle size of larger than 20 µm. Preferably, the particles in the suspension have a particle size distribution as follows: 3% to 25% of the particles have a particle size of smaller than 5 µm; 50% to 75% of the particles have a particle size of 5 µm to 20 µm; 10% to 15% of the particles have a particle size of 20 µm to 30 µm; and the remaining particles have a particle size of larger than 30 µm.

In some embodiments, the particles in the suspension have a particle size distribution as follows: not less than 5% of the particles have a particle size of smaller than 5 µm, and not less than 15% of the particles have a particle size of larger than 20 µm. Preferably, the particles in the suspension have a particle size distribution as follows: 15% to 25% of the particles have a particle size of smaller than 5 µm; 20% to 30% of the particles have a particle size of 5 µm to 10 µm; 25% to 30% of the particles have a particle size of 10 µm to 20 µm; 10% to 15% of the particles have a particle size of 20 µm to 30 µm; and the remaining particles have a particle size of larger than 30 µm. More preferably, the particles in the suspension have a particle size distribution as follows: 20% to 23% of the particles have a particle size of smaller than 5 µm; 23% to 27% of the particles have a particle size of 5 µm to 10 µm; 27% to 29% of the particles have a particle size of 10 µm to 20 µm; 13% to 15% of the particles have a particle size of 20 µm to 30 µm, and the remaining particles have a particle size of larger than 30 µm.

The preparation in the second aspect of the present invention is obtainable or obtained by the method in the first aspect of the present invention.

The long-acting brexpiprazole preparation for injection of the present invention is prepared using a solvent precipitation method, and the particle size of brexpiprazole may be controlled by regulating process parameters, wherein brexpiprazole, in an anhydrous form, may start to be released quickly and reach an effective therapeutic level after intramuscular injection, and continues to be released slowly to maintain a plasma drug concentration at the effective therapeutic level for 30 days or longer. After research, nitrogen protection during the process of dissolving brexpiprazole in an organic solvent can effectively prevent formation of a specific unknown impurity and improve the purity of a sample.

In addition, the sustained release effect of the long-acting brexpiprazole preparation for injection of the present invention can be achieved by controlling the particle size and dosage of brexpiprazole, independent of excipients and free from any polymer excipients such as poly(lactic-co-glycolic) acid or polylactic acid. Thus, the drug loading capacity is maximized, the injection volume is reduced, and the compliance of a patient is improved to the maximum extent.

### Brief Description of the Drawings

FIG. 1 shows the plasma drug concentration curves of a brexpiprazole suspension as a control preparation and the brexpiprazole suspensions of Example 2 and Example 15 in rats.
FIG. 2 shows the particle size distributions of the drug particles in the brexpiprazole suspensions of Example 2 and Example 15.
FIGS. 3 A to D shows the high performance liquid chromatograms of related substances of the brexpiprazole powders of Example 16 and Example 17.

### Detailed Description of the Invention

The present invention is further described below with reference to examples, which, however, are not intended to limit the scope of the present invention.

### Examples

### Example 1. Preparation of a brexpiprazole suspension

- Preparation of a brexpiprazole solution:
   10 g of brexpiprazole (anhydrous, self-made) was added with 100 mL of dimethyl sulfoxide, and stirred at 60°C for dissolution.
- Preparation of a precipitation solvent:
   200 mL of pure water and 0.1 g of Tween 80 were placed into a 500 mL beaker.
- Preparation of a sample:
   A shear head was placed into the precipitation solvent, and the linear velocity for shearing was regulated to 3.14 m/s. The brexpiprazole solution was pumped into the precipitation solvent at a velocity of 100 mL/min (with a pumping time of 36 s), and subjected to suction filtration, and the subsequent filtrate was removed. The filter cake was washed with 1000 mL of pure water, and the resulting solid was transferred into a drying oven at 40°C for drying to obtain dry powder.
- Preparation of a suspension:
   (i) Preparation of a dispersing agent: 2.5 g of sodium carboxymethyl cellulose, 0.2 g of sodium dihydrogen phosphate and 12.5 g of mannitol were placed into a 500 mL beaker, added with 249 mL of water, and stirred until completely dissolved.
   (ii) Dispersion of the sample: 5.7 g of the dry powder obtained in the sample preparation step was added with 44.3 g of the dispersing agent, and shaken up and down manually for about 5 min for uniform mixing to obtain the suspension.
- Measurement of particle size:
   The size of particles in the suspension was measured by a MasterSizer 3000 laser diffraction particle size analyzer (Malvern) with water as a measurement vehicle, and the average particle size Dv50 is 3.639 µm.

### Example 2. Preparation of a brexpiprazole suspension

- Preparation of a brexpiprazole solution:
   10 g of brexpiprazole was added with 100 mL of dimethyl sulfoxide, and stirred at 60°C for dissolution.
- Preparation of a precipitation solvent:
   200 mL of pure water and 0.1 g of Tween 80 were placed into a 500 mL beaker.
- Preparation of a sample:
   A shear head was placed into the precipitation solvent, and the linear velocity for shearing was regulated to 1.57 m/s. The brexpiprazole solution was pumped into the precipitation solvent at a velocity of 100 mL/min (with a pumping time of 60 s), and subjected to suction filtration, and the subsequent filtrate was removed. The filter cake was washed with 1000 mL of pure water, and the resulting solid was transferred into a drying oven at 40°C for drying to obtain dry powder.
- Preparation of a suspension:
   (i) Preparation of a dispersing agent: 0.1 g of Tween 80, 0.1 g of disodium hydrogen phosphate, 0.06 g of sodium dihydrogen phosphate and 0.9 g of sodium chloride were placed into a 250 mL beaker, added with 99 mL of water, and stirred until completely dissolved.
   (ii) Dispersion of the sample: 5.7 g of the dry powder obtained in the sample preparation step was added with 44.3 g of the dispersing agent, and shaken up and down manually for about 5 min for uniform mixing to obtain the suspension.
- Measurement of particle size:
   The size of particles in the suspension was measured by a MasterSizer 3000 laser diffraction particle size analyzer (Malvern) with water as a measurement vehicle, and the average particle size Dv50 is 10.617 µm.

### Example 3. Preparation of a brexpiprazole suspension

- Preparation of a brexpiprazole solution:
   20 g of brexpiprazole was added with 200 mL of dimethyl sulfoxide, and stirred at 60°C for dissolution.
- Preparation of a precipitation solvent:
   400 mL of pure water and 0.2 g of Tween 80 were placed into a 1000 mL beaker.
- Preparation of a sample:
   A stirring paddle was placed into the precipitation solvent, and the rotating velocity was regulated to 500 rpm. The brexpiprazole solution was pumped into the precipitation solvent at a velocity of 70 mL/min (with a pumping time of 120 s), and subjected to suction filtration, and the subsequent filtrate was removed. The filter cake was washed with 2000 mL of pure water, and the resulting solid was transferred into a drying oven at 40°C for drying to obtain dry powder.
- Preparation of a suspension:
   (i) Preparation of a dispersing agent: 0.1 g of Tween 80, 0.1 g of disodium hydrogen phosphate, 0.06 g of sodium dihydrogen phosphate and 0.9 g of sodium chloride were placed into a 250 mL beaker, added with 170 mL of water, and stirred until completely dissolved.
   (ii) Dispersion of the sample: 5.7 g of the dry powder obtained in the sample preparation step was added with 44.3 g of the dispersing agent, and shaken up and down manually for about 5 min for uniform mixing to obtain the suspension.
- Measurement of particle size:
   The size of particles in the suspension was measured by a MasterSizer 3000 laser diffraction particle size analyzer (Malvern) with water as a measurement vehicle, and the average particle size Dv50 is 10.848 µm.

### Example 4. Preparation of a brexpiprazole suspension

- Preparation of a brexpiprazole solution:
   15 g of brexpiprazole was added with 150 mL of dimethyl sulfoxide, and stirred at 60°C for dissolution.
- Preparation of a sample:
   A shear head was placed into the brexpiprazole solution, and the linear velocity for shearing was regulated to 4.71 m/s. Water at room temperature was pumped into the brexpiprazole solution at a velocity of 150 mL/min (with a pumping time of 60 s), and subjected to suction filtration, and the subsequent filtrate was removed. The filter cake was washed with 1500 mL of pure water and drained for later use.
- Preparation of a suspension:
   (i) Preparation of a dispersing agent 1: 0.2 g of Tween 80 was placed into a 250 mL beaker, added with 60 mL of water, and stirred until completely dissolved.
   (ii) Dispersion of a suspension intermediate sample: the filter cake obtained in the sample preparation step was added to the dispersing agent 1, and dispersed using a shearing machine at a linear velocity of 1.57 m/s to obtain the suspension intermediate sample, which was detected to have a drug content of 133.3%.
   (iii) Preparation of a dispersing agent 2 and uniform mixing of the sample: 0.6 g of sodium carboxymethyl cellulose, 0.3 g of Tween 80, 0.1 g of sodium dihydrogen phosphate and 8.1 g of mannitol were placed into a 50 mL beaker, added with 20 mL of water, and stirred until completely dissolved to obtain the dispersing agent 2. Subsequently, the dispersing agent 2 was added to the suspension intermediate sample obtained in step (ii), and stirred until uniformly mixed.
- Measurement of particle size:
   The size of particles in the suspension was measured by a MasterSizer 3000 laser diffraction particle size analyzer (Malvern) with water as a measurement vehicle, and the average particle size Dv50 is 19.495 µm.

### Example 5. Preparation of a brexpiprazole suspension

- Preparation of a brexpiprazole solution:
   15 g of brexpiprazole was added with 150 mL of dimethyl sulfoxide, and stirred at 60°C for dissolution.
- Preparation of a sample:
   A shear head was placed into a brexpiprazole solution, and the linear velocity for shearing was regulated to 4.71 m/s. Water at 4°C was pumped into the brexpiprazole solution at a velocity of 150 mL/min (with a pumping time of 60 s), and subjected to suction filtration, and the subsequent filtrate was removed. The filter cake was washed with 1500 mL of pure water, and the resulting solid was transferred into a drying oven at 40°C for drying to obtain dry powder.
- Preparation of a suspension:
   (i) Preparation of a dispersing agent: 1.7 g of sodium carboxymethyl cellulose, 0.4 g of Tween 80, 0.1 g of sodium dihydrogen phosphate and 8.3 g of mannitol were placed into a 250 mL beaker, added with 170 mL of water, and stirred until completely dissolved.
   (ii) Dispersion of the sample: 5.7 g of the dry powder obtained in the sample preparation step was added with 44.3 g of the dispersing agent, and shaken up and down manually for about 5 min for uniform mixing to obtain the suspension.
- Measurement of particle size:
   The size of particles in the suspension was measured by a MasterSizer 3000 laser diffraction particle size analyzer (Malvern) with water as a measurement vehicle, and the average particle size Dv50 is 17.021 µm.

### Example 6. Preparation of a brexpiprazole suspension

- Preparation of a brexpiprazole solution:
   15 g of brexpiprazole was added with 150 mL of dimethyl sulfoxide, and stirred at 60°C for dissolution.
- Preparation of a sample:
   A shear head was placed into a brexpiprazole solution, and the linear velocity for shearing was regulated to 10.99 m/s. Water at room temperature was pumped into the brexpiprazole solution at a velocity of 150 mL/min (with a pumping time of 60 s). The resulting suspension was further sheared at a shearing linear velocity of 10.99 m/s for 20 min and then subjected to suction filtration, and the subsequent filtrate was removed. The filter cake was washed with 1500 mL of pure water, and the resulting solid was transferred into a drying oven at 40°C for drying to obtain dry powder.
- Preparation of a suspension:
   (i) Preparation of a dispersing agent: 1.7 g of sodium carboxymethyl cellulose, 0.4 g of Tween 80, 0.1 g of sodium dihydrogen phosphate and 8.3 g of mannitol were placed into a 250 mL beaker, added with 170 mL of water, and stirred until completely dissolved.
   (ii) Dispersion of the sample: 5.7 g of the dry powder obtained in the sample preparation step was added with 44.3 g of the dispersing agent, and shaken up and down manually for about 5 min for uniform mixing to obtain the suspension.
- Measurement of particle size:
   The size of particles in the suspension was measured by a MasterSizer 3000 laser diffraction particle size analyzer (Malvern) with water as a measurement vehicle, and the average particle size Dv50 is 15.184 µm.

### Example 7. Preparation of a brexpiprazole suspension

- Preparation of a brexpiprazole solution:
   30 g of brexpiprazole was added with 300 mL of dimethyl sulfoxide, and stirred at 60°C for dissolution.
- Preparation of a precipitation solvent:
   100 mL of pure water, 100 mL of dimethyl sulfoxide and 0.1 g of Tween 80 were placed into a 500 mL beaker, and cooled to 20°C with an ice water bath.
- Preparation of a sample:
   A shear head was placed into the precipitation solvent, and the linear velocity for shearing was regulated to 1.57 m/s. The brexpiprazole solution was pumped into the precipitation solvent at a velocity of 100 mL/min (with a pumping time of 60 s), and subjected to suction filtration, and the subsequent filtrate was removed. The filter cake was washed with 1000 mL of pure water, and the resulting solid was transferred into a drying oven at 40°C for drying to obtain dry powder.
- Preparation of a suspension:
   (i) Preparation of a dispersing agent: 1.7 g of sodium carboxymethyl cellulose, 0.4 g of Tween 80, 0.1 g of sodium dihydrogen phosphate and 8.3 g of mannitol were placed into a 250 mL beaker, added with 170 mL of water, and stirred until completely dissolved.
   (ii) Dispersion of the sample: 5.7 g of the dry powder obtained in the sample preparation step was added with 44.3 g of the dispersing agent, and shaken up and down manually for about 5 min for uniform mixing to obtain the suspension.
- Measurement of particle size:
   The size of particles in the suspension was measured by a MasterSizer 3000 laser diffraction particle size analyzer (Malvern) with water as a measurement vehicle, and the average particle size Dv50 is 11.678 µm.

### Example 8. Preparation of brexpiprazole powder

- Preparation of a brexpiprazole solution:
   50 g of brexpiprazole was added with 500 mL of dimethyl sulfoxide, and stirred at 60°C for dissolution.
- Preparation of a precipitation solvent:
   0.7 g of Tween 80 was rinsed with 1400 mL of water for several times into a jacketed steel cup, and cooled to 15°C with a circulative cooling apparatus.
- Preparation of a sample:
   A shear head was placed into the precipitation solvent, and the linear velocity for shearing was regulated to 3.14 m/s. The brexpiprazole solution was pumped into the precipitation solvent at a velocity of 200 mL/min (with a pumping time of 2 min), and subjected to suction filtration, and the subsequent filtrate was removed. The filter cake was washed with 4000 mL of pure water, and the resulting solid was transferred into a drying oven at 40°C for drying to obtain dry powder.
- Measurement of particle size:
   The particle size was measured by a MasterSizer 3000 laser diffraction particle size analyzer (Malvern) with water as a measurement vehicle, and the average particle size Dv50 is 7.968 µm.

### Example 9. Preparation of brexpiprazole powder

- Preparation of a brexpiprazole solution:
   50 g of brexpiprazole was added with 500 mL of dimethyl sulfoxide, and stirred at 60°C for dissolution.
- Preparation of a precipitation solvent:
   0.7 g of Tween 80 was rinsed with 1400 mL of water for several times into a jacketed steel cup, and cooled to 5°C with a circulative cooling apparatus.
- Preparation of a sample:
   A shear head was placed into the precipitation solvent, and the linear velocity for shearing was regulated to 7.85 m/s. The brexpiprazole solution was pumped into the precipitation solvent at a velocity of 200 mL/min (with a pumping time of 2 min), and subjected to suction filtration, and the subsequent filtrate was removed. The filter cake was washed with 3000 mL of pure water, and the resulting solid was transferred into a drying oven at 40°C for drying to obtain dry powder.
- Measurement of particle size:
   The particle size was measured by a MasterSizer 3000 laser diffraction particle size analyzer (Malvern) with water as a measurement vehicle, and the average particle size Dv50 is 3.025 µm.

### Example 10. Preparation of brexpiprazole powder

- Preparation of a brexpiprazole solution:
   50 g of brexpiprazole was added with 500 mL of dimethyl sulfoxide, and stirred at 60°C for dissolution.
- Preparation of a precipitation solvent:
   0.6 g of Tween 80 was rinsed with 1200 mL of water for several times into a jacketed steel cup, and cooled to 10°C with a circulative cooling apparatus.
- Preparation of a sample:
   A shear head was placed into the precipitation solvent, and the linear velocity for shearing was regulated to 7.85 m/s. The brexpiprazole solution was pumped into the precipitation solvent at a velocity of 100 mL/min (with a pumping time of 4 min), and subjected to suction filtration, and the subsequent filtrate was removed. The filter cake was washed with 3000 mL of pure water, and the resulting solid was transferred into a drying oven at 40°C for drying to obtain dry powder.
- Measurement of particle size:
   The particle size was measured by a MasterSizer 3000 laser diffraction particle size analyzer (Malvern) with water as a measurement vehicle, and the average particle size Dv50 is 9.120 µm.

### Example 11. Preparation of brexpiprazole powder

- Preparation of a brexpiprazole solution:
   50 g of brexpiprazole was added with 500 mL of dimethyl sulfoxide, and stirred at 60°C for dissolution.
- Preparation of a precipitation solvent:
   0.6 g of Tween 80 was rinsed with 1200 mL water for several times into a jacketed steel cup, and cooled to 10°C with a circulative cooling apparatus.
- Preparation of a sample:
   A shear head was placed into the precipitation solvent, and the linear velocity for shearing was regulated to 7.85 m/s. The brexpiprazole solution was pumped into the precipitation solvent at a velocity of 200 mL/min (with a pumping time of 1.5 min), and subjected to suction filtration, and the subsequent filtrate was removed. The filter cake was washed with 3000 mL of pure water, and the resulting solid was transferred into a drying oven at 40°C for drying to obtain dry powder.
- Measurement of particle size:
   The particle size was measured by a MasterSizer 3000 laser diffraction particle size analyzer (Malvern) with water as a measurement vehicle, and the average particle size Dv50 is 8.785 µm.

### Example 12. Preparation of brexpiprazole powder

- Preparation of a brexpiprazole solution:
   50 g of brexpiprazole was added with 500 mL of dimethyl sulfoxide, and stirred at 60°C for dissolution.
- Preparation of a precipitation solvent:
   0.6 g of Tween 80 was rinsed with 1200 mL of anhydrous ethanol for several times into a jacketed steel cup, and cooled to -20°C with a circulative cooling apparatus.
- Preparation of a sample:
   A shear head was placed into the precipitation solvent, and the linear velocity for shearing was regulated to 7.85 m/s. The brexpiprazole solution was pumped into the precipitation solvent at a velocity of 200 mL/min (with a pumping time of 1.5 min), and subjected to suction filtration, and the subsequent filtrate was removed. The filter cake was washed with 3000 mL of pure water, and the resulting solid was transferred into a drying oven at 40°C for drying to obtain dry powder.
- Measurement of particle size:
   The particle size was measured by a MasterSizer 3000 laser diffraction particle size analyzer (Malvern) with water as a measurement vehicle, and the average particle size Dv50 is 17.856 µm.

### Example 13. Preparation of brexpiprazole powder

- Preparation of a brexpiprazole solution:
   50 g of brexpiprazole was added with 500 mL of dimethyl sulfoxide, and stirred at 60°C for dissolution.
- Preparation of a precipitation solvent:
   960 mL of pure water and 240 mL of anhydrous ethanol were placed into a jacketed steel cup, added with 0.6 g of Tween 80, and cooled to -10°C with a circulative cooling apparatus.
- Preparation of a sample:
   A shear head was placed into the precipitation solvent, and the linear velocity for shearing was regulated to 7.85 m/s. The brexpiprazole solution was pumped into the precipitation solvent at a velocity of 200 mL/min (with a pumping time of 1.5 min), and subjected to suction filtration, and the subsequent filtrate was removed. The filter cake was washed with 3000 mL of pure water, and the resulting solid was transferred into a drying oven at 40°C for drying to obtain dry powder.
- Measurement of particle size:
   The particle size was measured by a MasterSizer 3000 laser diffraction particle size analyzer (Malvern) with water as a measurement vehicle, and the average particle size Dv50 is 15.346 µm.

### Example 14. Preparation of brexpiprazole powder

- Preparation of a brexpiprazole solution:
   30 g of brexpiprazole was added with 300 mL of dimethyl sulfoxide, and stirred at 60°C for dissolution.
- Preparation of a precipitation solvent:
   120 mL of pure water and 80 mL of dimethyl sulfoxide were placed into a jacketed steel cup, added with 0.1 g of Tween 80, and cooled to 20°C with an ice water bath.
- Preparation of a sample:
   A shear head was placed into the precipitation solvent, and the linear velocity for shearing was regulated to 1.57 m/s. The brexpiprazole solution was pumped into the precipitation solvent at a velocity of 100 mL/min (with a pumping time of 1 min), and subjected to suction filtration, and the subsequent filtrate was removed. The filter cake was washed with 1000 mL of pure water, and the resulting solid was transferred into a drying oven at 40°C for drying to obtain dry powder.
- Measurement of particle size:
   The particle size was measured by a MasterSizer 3000 laser diffraction particle size analyzer (Malvern) with water as a measurement vehicle, and the average particle size Dv50 is 8.577 µm.

### Example 15 (Comparative Example). Preparation of a brexpiprazole suspension with ball milling method

- Preparation of a pre-ball-milled sample:
   2.5 g of sodium carboxymethyl cellulose, 0.4 g of sodium dihydrogen phosphate and 25.0 g of mannitol were added with 201 g of water, and stirred until completely dissolved. Subsequently, 68.4 g of brexpiprazole was added thereto and stirred uniformly such that the drug is completely wetted, thereby obtaining the pre-ball-milled sample.
- Preparation of a suspension sample:
   The pre-ball-milled sample was added to a ball mill for ball milling. The ball milling was performed under the following conditions: the zirconium bead size was 2.2 mm, the filling amount was 60%, the rotating velocity for ball milling was 1500 rpm, and the flow rate was 600 ml/min, with tap water being used as the cooling water. After the ball milling was completed, the drug solution was taken out, diluted with water to half of the original concentration, and then bottled.
- Measurement of particle size:
   The particle size was measured by a MasterSizer 3000 laser diffraction particle size analyzer (Malvern) with water as a measurement vehicle, and the average particle size Dv50 is 11.678 µm.

### Example 16. Preparation of brexpiprazole powder

- Preparation of a brexpiprazole solution:
   20 g of brexpiprazole was added with 200 mL of dimethyl sulfoxide, and stirred at 60°C for dissolution (no nitrogen bubbled thereto).
- Preparation of a precipitation solvent:
   200 mL of pure water was placed into a jacketed steel cup, added with 0.1 g of Tween 80, and cooled to 20°C with an ice water bath.
- Preparation of a sample:
   After brexpiprazole was completely dissolved, a shear head was placed into the precipitation solvent immediately, and the linear velocity for shearing was regulated to 2.35 m/s. The brexpiprazole solution was pumped into the precipitation solvent at a velocity of 100 mL/min (with a pumping time of 1 min), and subjected to suction filtration, and the subsequent filtrate was removed. The filter cake was washed with 1000 mL of pure water, and the resulting solid was transferred into a drying oven at 40°C for drying to obtain dry powder of Example 16-1.

After 4 hours of complete dissolution of brexpiprazole, a shear head was placed into the precipitation solvent, and the linear velocity for shearing was regulated to 2.35 m/s. The brexpiprazole solution was pumped into the precipitation solvent at a velocity of 100 mL/min (with a pumping time of 1 min), and subjected to suction filtration, and the subsequent filtrate was removed. The filter cake was washed with 1000 mL of pure water, and the resulting solid was transferred into a drying oven at 40°C for drying to obtain dry powder of Example 16-2.
- Determination of related substances:
   Determination was performed by high performance liquid chromatography (HPLC). Octadecylsilane chemically bonded silica was used as a filler (YMC-Pack Pro C18, 4.6 mm × 150 mm, 3 µm, or a chromatographic column with equivalent efficiency); 0.02 mol/L potassium dihydrogen phosphate solution (with a pH value regulated to 6.5 with 10% potassium hydroxide solution) was taken as mobile phase A; and acetonitrile-water (90:10) was taken as mobile phase B.

### Example 17. Preparation of Brexpiprazole Powder

- Preparation of a brexpiprazole solution:
   20 g of brexpiprazole was added with 200 mL of dimethyl sulfoxide, and stirred at 60°C for dissolution after nitrogen was bubbled thereto to completely dispel air.
- Preparation of a precipitation solvent:
   200 mL of pure water was placed into a jacketed steel cup, added with 0.1 g of Tween 80, and cooled to 20°C with an ice water bath.
- Preparation of a sample:
   After brexpiprazole was completely dissolved, a shear head was placed into the precipitation solvent immediately, and the linear velocity for shearing was regulated to 2.35 m/s. The brexpiprazole solution was pumped into the precipitation solvent at a velocity of 100 mL/min (with a pumping time of 1 min), and subjected to suction filtration, and the subsequent filtrate was removed. The filter cake was washed with 1000 mL of pure water, and the resulting solid was transferred into a drying oven at 40°C for drying to obtain dry powder of Example 17-1.

Under the protection of nitrogen, after 4 hours of complete dissolution of brexpiprazole, a shear head was placed into the precipitation solvent, and the linear velocity for shearing was regulated to 2.35 m/s. The brexpiprazole solution was pumped into the precipitation solvent at a velocity of 100 mL/min (with a pumping time of 1 min), and subjected to suction filtration, and the subsequent filtrate was removed. The filter cake was washed with 1000 mL of pure water, and the resulting solid was transferred into a drying oven at 40°C for drying to obtain dry powder of Example 17-2.
- Determination of related substances:
   Determination was performed by high performance liquid chromatography (HPLC). Octadecylsilane chemically bonded silica was used as a filler (YMC-Pack Pro C18, 4.6 mm × 150 mm, 3 µm, or a chromatographic column with equivalent efficiency); 0.02 mol/L potassium dihydrogen phosphate solution (with a pH value regulated to 6.5 with 10% potassium hydroxide solution) was taken as mobile phase A; and acetonitrile-water (90:10) was taken as mobile phase B.

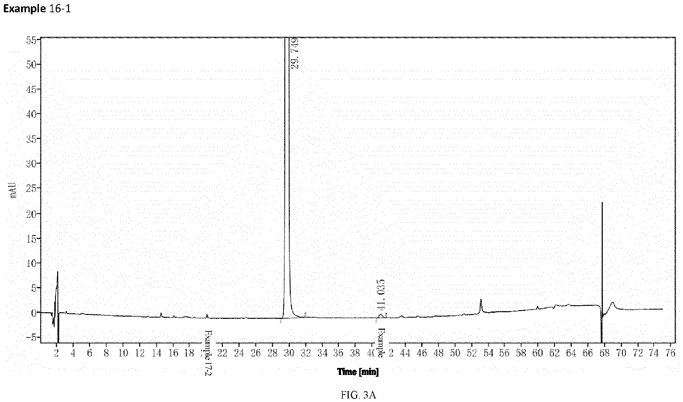

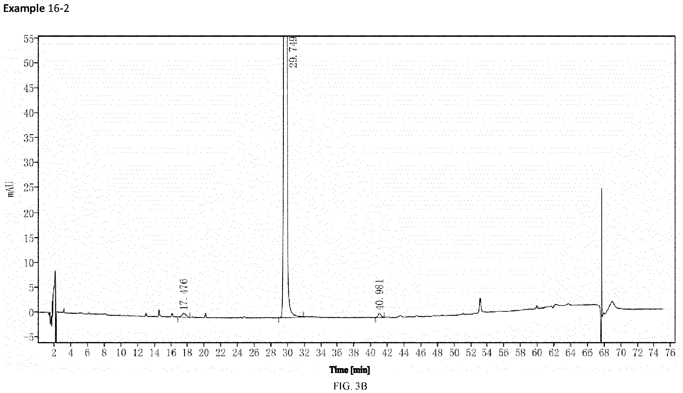

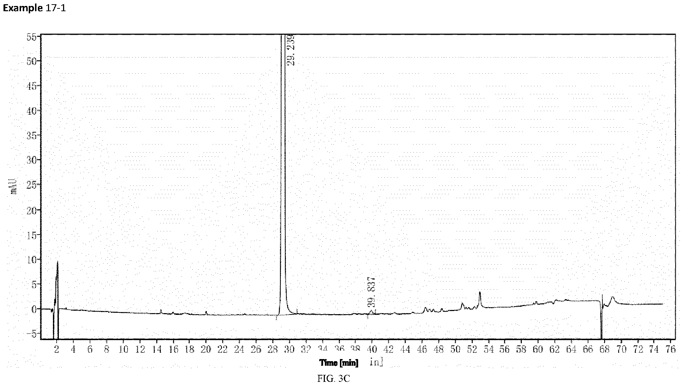

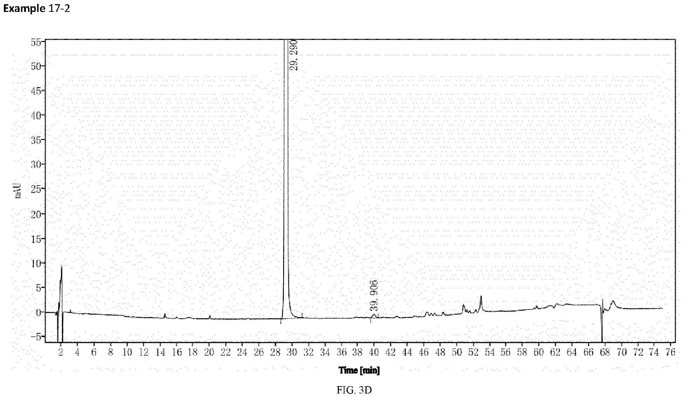

Table 1 and FIG. 3 show the test results of related substances in the four samples in Examples 16 and 17. It was found that introduction of nitrogen protection during the dissolution process of brexpiprazole could effectively prevent formation of an unknown impurity (RRT0.59). (RRT: relative retention time)

**Table 1. Results of Related Substances in Brexpiprazole Powders**

| Sample name | Content % | | | | | |
|---|---|---|---|---|---|---|
| | Known impurity A414S-Z6 (RRT0.54) | Unknown impurity 1 (RRT0.59) | Unknown impurity 2 (RRT0.68) | Unknown impurity 3 (RRT1.3 8) | Total impurities % | Number of impurities |
| Example 16-1 | Not detected | Not detected | Not detected | 0.05 | 0.05 | 1 |
| Example 16-2 | Not detected | 0.07 | Not detected | 0.05 | 0.12 | 2 |
| Example 17-1 | Not detected | Not detected | Not detected | Not detected | 0 | 0 |
| Example 17-2 | Not detected | Not detected | Not detected | 0.05 | 0.05 | 1 |

### Example 18. Preparation of a brexpiprazole suspension

- Preparation of a brexpiprazole solution:
   10 g of brexpiprazole (anhydrous, self-made) was added with 100 mL of dimethyl sulfoxide, and stirred at 60°C for dissolution after nitrogen was bubbled thereto.
- Preparation of a precipitation solvent:
   200 mL of pure water and 0.1 g of Tween 80 were placed into a 500 mL beaker.
- Preparation of a sample:
   A shear head was placed into the precipitation solvent, and the linear velocity for shearing was regulated to 3.14 m/s. The brexpiprazole solution was pumped into the precipitation solvent at a velocity of 100 mL/min (with a pumping time of 36 s), and subjected to suction filtration, and the subsequent filtrate was removed. The filter cake was washed with 1000 mL of pure water, and the resulting solid was transferred into a drying oven at 40°C for drying to obtain dry powder.
- Preparation of a suspension:
   (iii) Preparation of a dispersing agent: 2.5 g of sodium carboxymethyl cellulose, 0.2 g of sodium dihydrogen phosphate and 12.5 g of mannitol were placed into a 500 mL beaker, added with 249 mL of water, and stirred until completely dissolved.
   (iv) Dispersion of the sample: 5.7 g of the dry powder obtained in the sample preparation step was added with 44.3 g of the dispersing agent, and shaken up and down manually for about 5 min for uniform mixing to obtain the suspension.
- Measurement of particle size:
   The size of particles in the suspension was measured by a MasterSizer 3000 laser diffraction particle size analyzer (Malvern) with water as a measurement vehicle, and the average particle size Dv50 is 3.578 µm.

### Example 19. Preparation of a brexpiprazole suspension

- Preparation of a brexpiprazole solution:
   10 g of brexpiprazole was added with 100 mL of dimethyl sulfoxide, and stirred at 60°C for dissolution after nitrogen was bubbled thereto.
- Preparation of a precipitation solvent:
   200 mL of pure water and 0.1 g of Tween 80 were placed into a 500 mL beaker.
- Preparation of a sample:
   A shear head was placed into the precipitation solvent, and the linear velocity for shearing was regulated to 1.57 m/s. The brexpiprazole solution was pumped into the precipitation solvent at a velocity of 100 mL/min (with a pumping time of 60 s), and subjected to suction filtration, and the subsequent filtrate was removed. The filter cake was washed with 1000 mL of pure water, and the resulting solid was transferred into a drying oven at 40°C for drying to obtain dry powder.
- Preparation of a suspension:
   (iii) Preparation of a dispersing agent: 0.1 g of Tween 80, 0.1 g of disodium hydrogen phosphate, 0.06 g of sodium dihydrogen phosphate and 0.9 g of sodium chloride were placed into a 250 mL beaker, added with 99 mL of water, and stirred until completely dissolved.
   (iv) Dispersion of the sample: 5.7 g of the dry powder obtained in the sample preparation step was added with 44.3 g of the dispersing agent, and shaken up and down manually for about 5 min for uniform mixing to obtain the suspension.
- Measurement of particle size:
   The size of particles in the suspension was measured by a MasterSizer 3000 laser diffraction particle size analyzer (Malvern) with water as a measurement vehicle, and the average particle size Dv50 is 10.586 µm.

### Example 20. Preparation of a brexpiprazole suspension

- Preparation of a brexpiprazole solution:
   20 g of brexpiprazole was added with 200 mL of dimethyl sulfoxide, and stirred at 60°C for dissolution after nitrogen was bubbled thereto.
- Preparation of a precipitation solvent:
   400 mL of pure water and 0.2 g of Tween 80 were placed into a 1000 mL beaker.
- Preparation of a sample:
   A stirring paddle was placed into the precipitation solvent, and the rotating velocity was regulated to 500 rpm. The brexpiprazole solution was pumped into the precipitation solvent at a velocity of 70 mL/min (with a pumping time of 120 s), and subjected to suction filtration, and the subsequent filtrate was removed. The filter cake was washed with 2000 mL of pure water, and the resulting solid was transferred into a drying oven at 40°C for drying to obtain dry powder.
- Preparation of a suspension:
   (iii) Preparation of a dispersing agent: 0.1 g of Tween 80, 0.1 g of disodium hydrogen phosphate, 0.06 g of sodium dihydrogen phosphate and 0.9 g of sodium chloride were placed into a 250 mL beaker, added with 170 mL of water, and stirred until completely dissolved.
   (iv) Dispersion of the sample: 5.7 g of the dry powder obtained in the sample preparation step was added with 44.3 g of the dispersing agent, and shaken up and down manually for about 5 min for uniform mixing to obtain the suspension.
- Measurement of particle size:
   The size of particles in the suspension was measured by a MasterSizer 3000 laser diffraction particle size analyzer (Malvern) with water as a measurement vehicle, and the average particle size Dv50 is 10.294 µm.

### Example 21. Preparation of a brexpiprazole suspension

- Preparation of a brexpiprazole solution:
   15 g of brexpiprazole was added with 150 mL of dimethyl sulfoxide, and stirred at 60°C for dissolution after nitrogen was bubbled thereto.
- Preparation of a sample:
   A shear head was placed into a brexpiprazole solution, and the linear velocity for shearing was regulated to 4.71 m/s. Water at room temperature was pumped into the brexpiprazole solution at a velocity of 150 mL/min (with a pumping time of 60 s), and subjected to suction filtration, and the subsequent filtrate was removed. The filter cake was washed with 1500 mL of pure water and drained for later use.
- Preparation of a suspension:
   (iv) Preparation of a dispersing agent 1: 0.2 g of Tween 80 was placed into a 250 mL beaker, added with 60 mL of water, and stirred until completely dissolved.
   (v) Dispersion of a suspension intermediate sample: the filter cake obtained in the sample preparation step was added to the dispersing agent 1, and dispersed using a shearing machine at a linear velocity of 1.57 m/s to obtain the suspension intermediate sample, which was detected to have a drug content of 133.3%.
   (vi) Preparation of a dispersing agent 2 and uniform mixing of the sample: 0.6 g of sodium carboxymethyl cellulose, 0.3 g of Tween 80, 0.1 g of sodium dihydrogen phosphate and 8.1 g of mannitol were placed into a 50 mL beaker, added with 20 mL of water, and stirred until completely dissolved to obtain the dispersing agent 2. Subsequently, the dispersing agent 2 was added to the suspension intermediate sample obtained in step (ii), and stirred until uniformly mixed.
- Measurement of particle size:
   The size of particles in the suspension was measured by a MasterSizer 3000 laser diffraction particle size analyzer (Malvern) with water as a measurement vehicle, and the average particle size Dv50 is 19.418 µm.

### Example 22. Preparation of a brexpiprazole suspension

- Preparation of a brexpiprazole solution:
   15 g of brexpiprazole was added with 150 mL of dimethyl sulfoxide, and stirred at 60°C for dissolution after nitrogen was bubbled thereto.
- Preparation of a sample:
   A shear head was placed into a brexpiprazole solution, and the linear velocity for shearing was regulated to 4.71 m/s. Water at 4°C was pumped into the brexpiprazole solution at a velocity of 150 mL/min (with a pumping time of 60 s), and subjected to suction filtration, and the subsequent filtrate was removed. The filter cake was washed with 1500 mL of pure water, and the resulting solid was transferred into a drying oven at 40°C for drying to obtain dry powder.
- Preparation of a suspension:
   (iii) Preparation of a dispersing agent: 1.7 g of sodium carboxymethyl cellulose, 0.4 g of Tween 80, 0.1 g of sodium dihydrogen phosphate and 8.3 g of mannitol were placed into a 250 mL beaker, added with 170 mL of water, and stirred until completely dissolved.
   (iv) Dispersion of the sample: 5.7 g of the dry powder obtained in the sample preparation step was added with 44.3 g of the dispersing agent, and shaken up and down manually for about 5 min for uniform mixing to obtain the suspension.
- Measurement of particle size:
   The size of particles in the suspension was measured by a MasterSizer 3000 laser diffraction particle size analyzer (Malvern) with water as a measurement vehicle, and the average particle size Dv50 is 17.127 µm.

### Example 23. Preparation of a brexpiprazole suspension

- Preparation of a brexpiprazole solution:
   15 g of brexpiprazole was added with 150 mL of dimethyl sulfoxide, and stirred at 60°C for dissolution after nitrogen was bubbled thereto.
- Preparation of a sample:
   A shear head was placed into a brexpiprazole solution, and the linear velocity for shearing was regulated to 10.99 m/s. Water at room temperature was pumped into the brexpiprazole solution at a velocity of 150 mL/min (with a pumping time of 60 s). The resulting suspension was further sheared at a shearing linear velocity of 10.99 m/s for 20 min, and then subjected to suction filtration, and the subsequent filtrate was removed. The filter cake was washed with 1500 mL of pure water, and the resulting solid was transferred into a drying oven at 40°C for drying to obtain dry powder.
- Preparation of a suspension:
   (iii) Preparation of a dispersing agent: 1.7 g of sodium carboxymethyl cellulose, 0.4 g of Tween 80, 0.1 g of sodium dihydrogen phosphate and 8.3 g of mannitol were placed into a 250 mL beaker, added with 170 mL of water, and stirred until completely dissolved.
   (iv) Dispersion of the sample: 5.7 g of the dry powder obtained in the sample preparation step was added with 44.3 g of the dispersing agent, and shaken up and down manually for about 5 min for uniform mixing to obtain the suspension.
- Measurement of particle size:
   The size of particles in the suspension was measured by a MasterSizer 3000 laser diffraction particle size analyzer (Malvern) with water as a measurement vehicle, and the average particle size Dv50 is 15.035 µm.

### Example 24. Preparation of a brexpiprazole suspension

- Preparation of a brexpiprazole solution:
   30 g of brexpiprazole was added with 300 mL of dimethyl sulfoxide, and stirred at 60°C for dissolution after nitrogen was bubbled thereto.
- Preparation of a precipitation solvent:
   100 mL of pure water, 100 mL of dimethyl sulfoxide and 0.1 g of Tween 80 were placed into a 500 mL beaker, and cooled to 20°C with an ice water bath.
- Preparation of a sample:
   A shear head was placed into the precipitation solvent, and the linear velocity for shearing was regulated to 1.57 m/s. The brexpiprazole solution was pumped into the precipitation solvent at a velocity of 100 mL/min (with a pumping time of 60 s), and subjected to suction filtration, and the subsequent filtrate was removed. The filter cake was washed with 1000 mL of pure water, and the resulting solid was transferred into a drying oven at 40°C for drying to obtain dry powder.
- Preparation of a suspension:
   (iii) Preparation of a dispersing agent: 1.7 g of sodium carboxymethyl cellulose, 0.4 g of Tween 80, 0.1 g of sodium dihydrogen phosphate and 8.3 g of mannitol were placed into a 250 mL beaker, added with 170 mL of water, and stirred until completely dissolved .
   (iv) Dispersion of the sample: 5.7 g of the dry powder obtained in the sample preparation step was added with 44.3 g of the dispersing agent, and shaken up and down manually for about 5 min for uniform mixing to obtain the suspension.
- Measurement of particle size:
   The size of particles in the suspension was measured by a MasterSizer 3000 laser diffraction particle size analyzer (Malvern) with water as a measurement vehicle, and the average particle size Dv50 is 11.766 µm.

### Example 25. Preparation of brexpiprazole powder

- Preparation of a brexpiprazole solution:
   50 g of brexpiprazole was added with 500 mL of dimethyl sulfoxide, and stirred at 60°C for dissolution after nitrogen was bubbled thereto.
- Preparation of a precipitation solvent:
   0.7 g of Tween 80 was rinsed with 1400 mL of water for several times into a jacketed steel cup, and cooled to 15°C with a circulative cooling apparatus.
- Preparation of a sample:
   A shear head was placed into the precipitation solvent, and the linear velocity for shearing was regulated to 3.14 m/s. The brexpiprazole solution was pumped into the precipitation solvent at a velocity of 200 mL/min (with a pumping time of 2 min), and subjected to suction filtration, and the subsequent filtrate was removed. The filter cake was washed with 4000 mL of pure water, and the resulting solid was transferred into a drying oven at 40°C for drying to obtain dry powder.
- Measurement of particle size:
   The particle size was measured by a MasterSizer 3000 laser diffraction particle size analyzer (Malvern) with water as a measurement vehicle, and the average particle size Dv50 is 7.796 µm.

### Example 26. Preparation of brexpiprazole powder

- Preparation of a brexpiprazole solution:
   50 g of brexpiprazole was added with 500 mL of dimethyl sulfoxide, and stirred at 60°C for dissolution after nitrogen was bubbled thereto.
- Preparation of a precipitation solvent:
   0.7 g of Tween 80 was rinsed with 1400 mL of water for several times into a jacketed steel cup, and cooled to 5°C with a circulative cooling apparatus.
- Preparation of a sample:
   A shear head was placed into the precipitation solvent, and the linear velocity for shearing was regulated to 7.85 m/s. The brexpiprazole solution was pumped into the precipitation solvent at a velocity of 200 mL/min (with a pumping time of 2 min), and subjected to suction filtration, and the subsequent filtrate was removed. The filter cake was washed with 3000 mL of pure water, and the resulting solid was transferred into a drying oven at 40°C for drying to obtain dry powder.
- Measurement of particle size:
   The particle size was measured by a MasterSizer 3000 laser diffraction particle size analyzer (Malvern) with water as a measurement vehicle, and the average particle size Dv50 is 3.134 µm.

### Example 27. Preparation of brexpiprazole powder

- Preparation of a brexpiprazole solution:
   50 g of brexpiprazole was added with 500 mL of dimethyl sulfoxide, and stirred at 60°C for dissolution after nitrogen was bubbled thereto.
- Preparation of a precipitation solvent:
   0.6 g of Tween 80 was rinsed with 1200 mL water for several times into a jacketed steel cup, and cooled to 10°C with a circulative cooling apparatus.
- Preparation of a sample:
   A shear head was placed into the precipitation solvent, and the linear velocity for shearing was regulated to 7.85 m/s. The brexpiprazole solution was pumped into the precipitation solvent at a velocity of 100 mL/min (with a pumping time of 4 min), and subjected to suction filtration, and the subsequent filtrate was removed. The filter cake was washed with 3000 mL of pure water, and the resulting solid was transferred into a drying oven at 40°C for drying to obtain dry powder.
- Measurement of particle size:
   The particle size was measured by a MasterSizer 3000 laser diffraction particle size analyzer (Malvern) with water as a measurement vehicle, and the average particle size Dv50 is 9.021 µm.

### Example 28. Preparation of brexpiprazole powder

- Preparation of a brexpiprazole solution:
   50 g of brexpiprazole was added with 500 mL of dimethyl sulfoxide, and stirred at 60°C for dissolution after nitrogen was bubbled thereto.
- Preparation of a precipitation solvent:
   0.6 g of Tween 80 was rinsed with 1200 mL water for several times into a jacketed steel cup, and cooled to 10°C with a circulative cooling apparatus.
- Preparation of a sample:
   A shear head was placed into the precipitation solvent, and the linear velocity for shearing was regulated to 7.85 m/s. The brexpiprazole solution was pumped into the precipitation solvent at a velocity of 200 mL/min (with a pumping time of 1.5 min), and subjected to suction filtration, and the subsequent filtrate was removed. The filter cake was washed with 3000 mL of pure water, and the resulting solid was transferred into a drying oven at 40°C for drying to obtain dry powder.
- Measurement of particle size:
   The particle size was measured by a MasterSizer 3000 laser diffraction particle size analyzer (Malvern) with water as a measurement vehicle, and the average particle size Dv50 is 8.702 µm.

### Example 29. Preparation of brexpiprazole powder

- Preparation of a brexpiprazole solution:
   50 g of brexpiprazole was added with 500 mL of dimethyl sulfoxide, and stirred at 60°C for dissolution after nitrogen was bubbled thereto.
- Preparation of a precipitation solvent:
   0.6 g of Tween 80 was rinsed with 1200 mL of anhydrous ethanol for several times into a jacketed steel cup, and cooled to -20°C with a circulative cooling apparatus.
- Preparation of a sample:
   A shear head was placed into the precipitation solvent, and the linear velocity for shearing was regulated to 7.85 m/s. The brexpiprazole solution was pumped into the precipitation solvent at a velocity of 200 mL/min (with a pumping time of 1.5 min), and subjected to suction filtration, and the subsequent filtrate was removed. The filter cake was washed with 3000 mL of pure water, and the resulting solid was transferred into a drying oven at 40°C for drying to obtain dry powder.
- Measurement of particle size:
   The particle size was measured by a MasterSizer 3000 laser diffraction particle size analyzer (Malvern) with water as a measurement vehicle, and the average particle size Dv50 is 17.742 µm.

### Example 30. Preparation of brexpiprazole powder

- Preparation of a brexpiprazole solution:
   50 g of brexpiprazole was added with 500 mL of dimethyl sulfoxide, and stirred at 60°C for dissolution after nitrogen was bubbled thereto.
- Preparation of a precipitation solvent:
   960 mL of pure water and 240 mL of anhydrous ethanol were placed into a jacketed steel cup, added with 0.6 g of Tween 80, and cooled to -10°C with a circulative cooling apparatus.
- Preparation of a sample:
   A shear head was placed into the precipitation solvent, and the linear velocity for shearing was regulated to 7.85 m/s. The brexpiprazole solution was pumped into the precipitation solvent at a velocity of 200 mL/min (with a pumping time of 1.5 min), and subjected to suction filtration, and the subsequent filtrate was removed. The filter cake was washed with 3000 mL of pure water, and the resulting solid was transferred into a drying oven at 40°C for drying to obtain dry powder.
- Measurement of particle size:
   The particle size was measured by a MasterSizer 3000 laser diffraction particle size analyzer (Malvern) with water as a measurement vehicle, and the average particle size Dv50 is 15.468 µm.

### Example 31. Preparation of brexpiprazole powder

- Preparation of a brexpiprazole solution:
   30 g of brexpiprazole was added with 300 mL of dimethyl sulfoxide, and stirred at 60°C for dissolution after nitrogen was bubbled thereto.
- Preparation of a precipitation solvent:
   120 mL of pure water and 80 mL of dimethyl sulfoxide were placed into a jacketed steel cup, added with 0.1 g of Tween 80, and cooled to 20°C with an ice water bath.
- Preparation of a sample:
   A shear head was placed into the precipitation solvent, and the linear velocity for shearing was regulated to 1.57 m/s. The brexpiprazole solution was pumped into the precipitation solvent at a velocity of 100 mL/min (with a pumping time of 1 min), and subjected to suction filtration, and the subsequent filtrate was removed. The filter cake was washed with 1000 mL of pure water, and the resulting solid was transferred into a drying oven at 40°C for drying to obtain dry powder.
- Measurement of particle size:
   The particle size was measured by a MasterSizer 3000 laser diffraction particle size analyzer (Malvern) with water as a measurement vehicle, and the average particle size Dv50 is 8.321 µm.

### Example 32. Plasma concentration of brexpiprazole in SD rats after intramuscular injection of brexpiprazole suspensions

Experimental animals: 12 SD rats (male) were randomly divided into 3 groups (n = 4) according to body weight, denoted as group A, group B and group C.

Control preparation: a brexpiprazole suspension (2 mg/mL), which was prepared by adding 20 g of water to 0.4 g of sodium carboxymethyl cellulose, stirring for dissolution, then adding 0.04 g of brexpiprazole, and stirring until the drug was uniformly dispersed.

Administration manner and dose:
Group A: The brexpiprazole suspension control preparation, 10 mg/kg, oral gavage.
Group B: The brexpiprazole suspension of Example 2, 30 mg/kg, intramuscular injection in lateral thighs.
Group C: The brexpiprazole suspension of Example 15, 30 mg/kg, intramuscular injection in lateral thighs.

Sampling and detection: 0.25 ml of blood (with EDTA-K2 anticoagulation) was sampled from tail veins before administration (0 h), at 0.5 h, 1 h, 1.5 h, 2 h, 3 h, 4 h, 6 h and 8 h on the day of administration, on Day 1 (24 h), on Day 2 (48 h), on Day 3 (72 h), on Day 4 (96 h), on Day 5 (120 h), on Day 6 (144 h), on Day 7 (168 h), on Day 9 (216 h), on Day 11 (264 h), on Day 13 (312 h), on Day 15 (360 h), on Day 17 (408 h), on Day 19 (456 h), on Day 21 (504 h), on Day 25 (600 h), on Day 29 (696 h), on Day 36 (864 h), on Day 42 (1008 h), on Day 49 (1176 h), on Day 56 (1344 h), and on Day 63 (1512 h) after administration for group B and group C; and 0.25 ml of blood (with EDTA-K2 anticoagulation) was sampled from veins before administration (0 h), at 0.5 h, 1 h, 1.5 h, 2 h, 3 h, 4 h, 6 h and 8 h on the day of administration, on Day 1 (24 h), and on Day 2 (48 h) after administration for group A. The collected whole blood was temporarily stored at room temperature, and centrifuged at 4000 rpm for 10 min within 1 h to separate plasma (4°C). The collected plasma was stored at a -80°C refrigerator for a later detection. The plasma samples after administration were detected, and a drug-time curve was fitted with the measured plasma drug concentration, to calculate pharmacokinetic parameters.

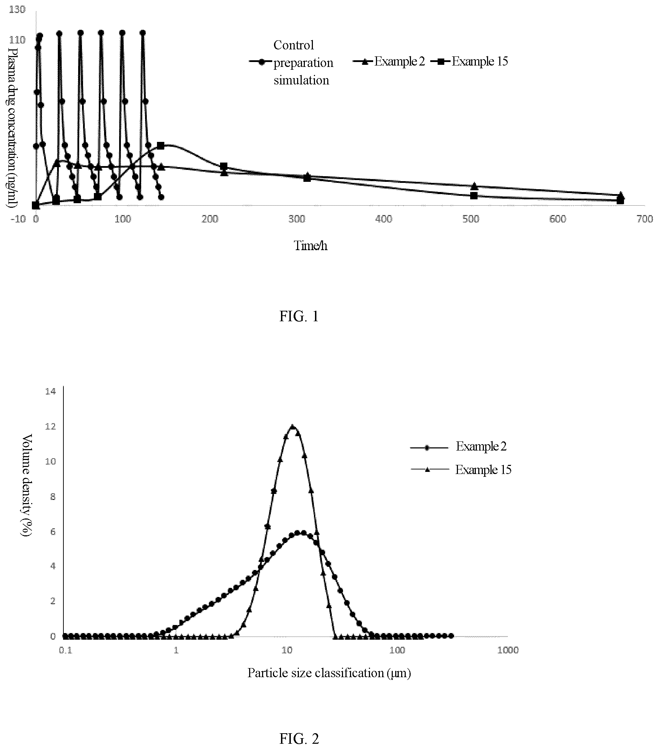

FIG. 1 shows a curve of the concentration of brexpiprazole in rat plasma over time. It can be seen from FIG. 1 that both the samples in Example 2 and Example 15 could achieve the effect of sustained release for 30 days. However, the suspension prepared by the ball milling method in Example 15 was released slowly in the rats at the early stage, and had a great fluctuation of a plasma drug concentration, and a smooth and effective plasma drug concentration could be hardly maintained. In contrast, the suspension prepared by the solvent precipitation method in Example 2 was quick in effect and stable in release, and had a gentle trend of the plasma drug concentration, which is more conducive to clinical administration.

### Example 33. Investigation of particle size distribution and specific surface area of brexpiprazole suspensions

The specific surface areas of the particles in the suspensions of Example 2 and Example 15 were measured (instrument: full automatic specific surface area and porosity analyzer BET, model: Micromeritics ASAP2460). The results showed that the specific surface area of the particles in the suspension of Example 2 was 2.0 m²/g, and the specific surface area of the particles in the suspension of Example 15 was 1.0 m²/g. Due to the larger specific surface area, the particles in the suspension of Example 2 had a quicker release at the early stage.

In addition, the particle size distributions of the drug in the brexpiprazole suspensions of Example 2 and Example 15 are shown in FIG. 2 and Table 2. It can be seen that the particles in the suspension of Example 15 had a very narrow particle size distribution with the particle size mainly concentrated in 5-20 µm, and lacked particles with a small particle size and particles with a large particle size compared with Example 2. This was the reason for slow release at the early stage and quick release at the later stage in vivo for Example 15. Meanwhile, the results of the particle size distributions of the samples in Example 3, Example 6, Example 23 and Example 24 are also listed in the following table. It can be seen that the samples prepared by the solvent precipitation method had a wider particle size distribution, and could be quickly released in vivo as having leading edge peaks; and meanwhile, the contained particles with a large particle size can be slowly released to maintain an effective plasma drug concentration in vivo.

**Table 2. Particle Size Distribution of the Drug in Brexpiprazole Suspensions**

| | 0-5 (µm) | 5-10 (µm) | 10-20 (µm) | 20-30 (µm) | >30 (µm) |
|---|---|---|---|---|---|
| Example 2 | 21.77% | 25.05% | 28.7% | 14.26% | 10.22% |
| Example 15 | 1.01% | 33.6% | 53.92% | 11.46% | 0.01% |
| Example 3 | 16.18% | 28.12% | 38.64% | 10.3% | 6.76% |
| Example 6 | 13.59% | 22.34% | 37.25% | 13.87% | 12.95% |
| Example 23 | 13.93% | 23.78% | 39.05% | 12.14% | 11.1% |
| Example 24 | 18.44% | 23.54% | 35.17% | 13.68% | 9.17% |

## Claims

1. A method for preparing a long-acting brexpiprazole preparation for injection, comprising the following steps:
(a) dissolving brexpiprazole in an organic solvent to form a brexpiprazole solution, wherein the organic solvent is selected from a group consisting of dimethyl sulfoxide, dichloromethane, dichloromethane-methanol, dimethylformamide, dimethylformamide-isopropanol, dimethylformamide-water, tetrahydrofuran-water, tetrahydrofuran-isopropanol, ethanol, and glacial acetic acid-water, preferably selected from a group consisting of dimethylsulfoxide, dichloromethane, dichloromethane-methanol, ethanol and glacial acetic acid-water;
(b1) pumping the brexpiprazole solution into a precipitation solvent under shearing or vigorous stirring to precipitate brexpiprazole; or (b2) pumping a precipitation solvent into the brexpiprazole solution under shearing or vigorous stirring to precipitate brexpiprazole, wherein the precipitation solvent is selected from a group consisting of water, an organic solvent selected from a group consisting of ethanol and dimethyl sulfoxide, and a mixture thereof, and optionally comprises a surfactant selected from a group consisting of Tween 20, Tween 80, Span 20, Span 40, Span 60, Span 80, succinate and glyceryl monostearate;
(c) filtering, and optionally washing a resulting solid with water in an amount of not less than 5 times, preferably 8 to 20 times, and more preferably 10 to 15 times a volume of the brexpiprazole solution in step (b1) or (b2); and
(d1) drying the solid, and packaging the dried solid and an excipient solution separately to obtain the long-acting brexpiprazole preparation for injection; or (d2) dispersing the solid into an excipient solution after drying or directly without drying to obtain the long-acting brexpiprazole preparation for injection.

2. The method according to claim 1, wherein step (a) is performed under nitrogen protection.

3. The method according to claim 1 or 2, wherein the organic solvent in step (a) is dimethyl sulfoxide; and the dissolving is performed with stirring at a temperature of 40°C to 80°C, preferably 50°C to 70°C, more preferably 60°C to 70°C, and more preferably about 60°C to 65°C.

4. The method according to any one of claims 1 to 3, wherein in step (b1) or (b2), the precipitation solvent is water or ethanol, or water-ethanol or water-dimethyl sulfoxide in a volume ratio of 20:1 to 1:20, preferably 10:1 to 1:10, and more preferably 5:1 to 1:5; and the precipitation solvent comprises Tween 80 in an amount of 0.1% to 3%, preferably 0.5% to 2%, of a weight of brexpiprazole in a resulting system after the completion of the pumping.

5. The method according to any one of claims 1 to 4, wherein in step (b1) or (b2), the pumping is performed by a single pump head or a plurality of pump heads; each pump head is at a pumping velocity of 70 mL/min to 300 mL/min, preferably 70 mL/min to 220 mL/min, and preferably 100 mL/min to 200 mL/min, such as 150 mL/min; and a volume ratio of the brexpiprazole solution to the precipitation solvent is 2:1 to 1:10, preferably 2:1 to 1:5, and more preferably 1:1 to 1:4, such as 1:2.

6. The method according to any one of claims 1 to 5, wherein in step (b1) or (b2), the precipitation solvent is at a temperature of -20°C to 25°C, preferably 0°C to 20°C.

7. The method according to any one of claims 1 to 6, wherein in step (b1) or (b2), the vigorous stirring is at a velocity of 200 rpm to 2000 rpm, preferably 500 rpm to 1000 rpm; and the shearing is at a linear velocity of 1.57 m/s to 15.7 m/s.

8. The method according to claim 7, wherein in step (b1), the shearing is at a linear velocity of 1.57 m/s to 15.7 m/s, preferably 1.57 m/s to 4.71 m/s, and in step (b2), the shearing is at a linear velocity of 7.85 m/s to 12.56 m/s.

9. The method according to any one of claims 1 to 7, wherein the resulting solid in step (c) does not contain a particle binder such as sodium chloride.

10. A long-acting brexpiprazole preparation for injection, which is a suspension, or can be prepared as a suspension by mixing a solid contained in the preparation and an excipient solution, wherein the suspension contains 5 wt% to 40 wt%, preferably 5 wt% to 20 wt%, and more preferably 7 wt% to 17 wt% of brexpiprazole.

11. The preparation according to claim 10, wherein particles in the suspension have a particle size distribution as follows: not less than 3% of the particles have a particle size of smaller than 5 µm, and not less than 15% of the particles have a particle size of larger than 20 µm.

12. The preparation according to claim 11, wherein the particles in the suspension have a particle size distribution as follows: 3% to 25% of the particles have a particle size of smaller than 5 µm; 50% to 75% of the particles have a particle size of 5 µm to 20 µm; 10% to 15% of the particles have a particle size of 20 µm to 30 µm; and the remaining particles have a particle size of larger than 30 µm.

13. The preparation according to claim 10, wherein the particles in the suspension have a particle size distribution as follows: not less than 5% of the particles have a particle size of smaller than 5 µm, and not less than 15% of the particles have a particle size of larger than 20 µm.

14. The preparation according to claim 13, wherein the particles in the suspension have a particle size distribution as follows: 15% to 25% of the particles have a particle size of smaller than 5 µm; 20% to 30% of the particles have a particle size of 5 µm to 10 µm; 25% to 30% of the particles have a particle size of 10 µm to 20 µm; 10% to 15% of the particles have a particle size of 20 µm to 30 µm; and the remaining particles have a particle size of larger than 30 µm.

15. The preparation according to any one of claims 10 to 14, which is obtainable or obtained by the method according to any one of claims 1 to 9.
